# EUROPEAN PATENT SPECIFICATION

(11) **EP 1 475 387 B1**
(45) Date of publication and mention of the grant of the patent: **15.11.2006**
(21) Application number: 04251544.5
(22) Date of filing: 18.03.2004
(51) Int. Cl.: C07K 1/30, G01N 33/68

(54) **Method for preparing assay samples**
Verfahren zur Bereitstellung von Proben zum Nachweis
Procédé de préparation d'échantillons pour le dépistage

(30) Priority: 20.03.2003 US 393672
(43) Date of publication of application: 10.11.2004
(73) Proprietor: Bayer HealthCare LLC, Tarrytown, New York 10591 (US)
(72) Inventor: Quinn, John J., Concord, California 94521 (US); Piran, Uri, Sharon, Massachusetts 02067 (US); Livshin, Laurie A., Ardsley, New York 10502 (US); Yannoni, Claudine Z., Newtownville, Massachusetts 02460 (US)
(74) Representative: Mallalieu, Catherine Louise

(56) References cited:
- WO-A-95/33827
- WO-A-02/055727
- US-A- 5 681 946
- DATABASE WPI 1996, Derwent Publications Ltd., London, GB; AN 1996-336568 XP002313501 & JP 08 154678 A (HITACHI DENSHI ENG KK) 18 June 1996 (1996-06-18)

## Description

### FIELD OF THE INVENTION

This invention relates to a method for preparing a sample for use in an assay. More particularly, the invention relates to the use of a polyalkylene glycol and magnetic particles, and a salt concentration of less than or equal to about 0.2M, to prepare samples for subsequent use in assays such as hybridization assays and immunoassays.

### BACKGROUND ART

Magnetic particles coupled to specific binding molecules have been used for separation of small molecules, macromolecules, viruses, bacteria and cells from liquids, by a process of specific binding. Magnetic particles without immobilized specific binders have also been used to separate nucleic acids and viruses, by a process of nonspecific adsorption (Lyle J. Arnold, Jr. et al., EP 0 281 390 B1, 1994; Bitton et al., "Removal of Escherichia coli bacteriophage T7 by Magnetic Filtration," *Water Research* **8**:549 (1974); Warren, "New purification procedure for biological vaccines," *Immunization Jap. Encephalitis conf,* Hammon (Ed.). Williams & Wilkins. Baltimore MD pp. 152-154 (1972)). Magnetic particles have the advantage of settling under gravity, while application of a magnetic field serves to expedite this already rapid process. Thus, separation can take place in 1-2 minutes instead of the typical hour-long centrifugation procedure. Unfortunately, nonspecific adsorption is too weak and unreliable when used with biological samples, due to competition on the nonspecific binding sites by undesirable macromolecules and small molecules.

Polyalkylene glycols such as polyethylene glycol (PEG) have been extensively used for separating proteins and viruses from biological samples (Fried et al., *Enzymology* 22:238 (1971). PEG precipitates proteins by binding water and excluding water that keeps proteins in solution. The type of precipitated protein is determined by the PEG concentration. At low concentrations, such as 4% w/v in combination with one to two molar salt, viruses and bacteriophages precipitate. This is a standard method for separation and concentration of these microorganisms. This process usually also requires an incubation at 4°C and subsequent centrifugation to sediment the precipitate (Yamamoto et al., *Virology* 40:734 (1971)). WO 2002/055727 teaches the purification of nucleic acids from a sample using PEG as a single reagent.

The combination of magnetic particles and PEG is not common, although it has been suggested for enhanced separation of proteins (Munro et al., *Biotechnology Letters* 3:297 (1981)). The combination is also described in US Patent No. 5,681,946 to Reeve for the nonspecific binding of bacteriophage and viruses, and typically uses a high salt concentrations (e.g., 2.5M sodium chloride). The combination is also described in US Patent No. 5,665,554 to Reeve et al. as a nucleic acid precipitation reagent (e.g., polyethylene glycol in aqueous sodium chloride) for the recovery of a material such as plasmid DNA, from a solution containing genomic DNA and cellular debris. It appears that precipitated bacteriophage, viruses and plasmid DNA readily adsorb in a nonspecific mechanism to magnetic particles, thus allowing the more rapid and convenient magnetic separation to replace standard centrifugation techniques. The aforementioned separations have been done for preparative but not for analytical purposes.

In spite of the advances in the art, there continues to be a need for improved analytical methods. In particular, there is a need to provide for more rapid and accurate testing and analysis methods. The present invention addresses those needs by providing a method for preparing samples for use in assays. The present invention involves the use of specific reagent combinations of a polyalkylene glycol and magnetic particles that find particular utility in analytical applications such as the preparation of samples to be analyzed in immunoassays or hybridization assays. Unlike the polyalkylene glycol solutions used in previous nucleic acid and protein separations, the instant invention uses a low ionic strength buffer. Surprisingly, it was found that both the minimization and omission of the salt gave better assay results, e.g., at high salt concentrations the precipitate did not adhere to magnetic particles. In addition, the reduced salt concentration provides for a universal sample that can be used in a variety of assays.

### DISCLOSURE OF THE INVENTION

One aspect of the invention relates to a method for preparing a sample for use in an assay comprising: (a) adding a polyalkylene glycol and magnetic particles to a sample to form a mixture, wherein the magnetic particles are capable of non-specific binding to protein-containing materials contained within the mixture and wherein the mixture has a salt concentration of less than or equal to about 0.2M; (b) precipitating the protein-containing materials out of the mixture when the protein-containing materials become non-specifically bound to the magnetic particles, to form a precipitate and a supernatant; (c) discarding the supernatant; and (d) isolating the precipitate to produce a prepared sample.

One aspect of the invention relates to a method for preparing a blood sample for use in an assay comprising: (a) adding a polyalkylene glycol and magnetic particles to a blood sample to form a mixture, wherein the magnetic particles are capable of non-specific binding to cells contained within the mixture and wherein the mixture has a salt concentration of less than or equal to about 0.2M; (b) precipitating the cells out of the mixture when the cells become non-specifically bound to the magnetic particles, to form a precipitate and a supernatant; (c) discarding the supernatant; and (d) isolating the precipitate to produce a prepared blood sample.

Another aspect of the invention pertains to a kit useful in preparing samples for use in an assay comprising: (a) a polyalkylene glycol; (b) magnetic particles that are capable of non-specific binding to protein-containing materials contained within the samples; (c) salt having a concentration of less than or equal to about 0.2M; and (d) instructions for preparing the samples.

### DETAILED DESCRIPTION OF THE INVENTION

The present invention comprises a method and kits useful in the preparation of samples for use in a variety of assays. The addition of a polyalkylene glycol and magnetic particles to a blood sample, under low salt concentration conditions (salt concentration of less than or equal to about 0.2M), provides for non-specific binding of cells contained within the sample solution, and subsequent precipitation of the bound cells. The supernatant is discarded and the precipitate isolated and either used immediately in an assay or stored for subsequent use, under appropriate storage conditions.

One of the advantages of this preparatory method is that it provides for a rapid means of preparing samples. By using the methods described herein, samples can be prepared in a matter of minutes, as compared to common centrifugation techniques that can take up to one hour or more, and eliminate the need for costly centrifugation equipment. Another advantage is that magnetic separation techniques are more readily automated.

Before describing detailed embodiments of the invention, it will be useful to set forth definitions that are used in describing the invention. The definitions set forth apply only to the terms as they are used in this patent and may not be applicable to the same terms as used elsewhere, for example in scientific literature or other patents or applications including other applications by these inventors or assigned to common owners. The following description of the preferred embodiments and examples are provided by way of explanation and illustration. As such, they are not to be viewed as limiting the scope of the invention as defined by the claims. Additionally, when examples are given, they are intended to be exemplary only and not to be restrictive. For example, when an example is said to "include" a specific feature, that is intended to imply that it may have that feature but not that such examples are limited to those that include that feature.

It must be noted that, as used in this specification and the appended claims, the singular forms "a", "an" and "the" include plural referents unless the context clearly dictates otherwise. Thus, for example, reference to "a material" includes a mixture of two or more such materials, reference to "a detergent" includes mixtures of two or more such detergents, and the like.

In describing and claiming the present invention, the following terminology will be used in accordance with the definitions set out below.

"Assay" is used herein to mean an *in vitro* test conducted on a biological sample (typically blood) to measure the presence (qualitative) or concentration (quantitative) of a given analyte (material of interest) *in vitro*.

"Cell" is used herein to mean cells that are typically found in blood, as well as those found in patients infected with a viral or bacterial organism. These include, by way of illustration and not limitation, erythrocytes (red blood cells); leukocytes (white blood cells) including polymorphonuclear leukocytes (neutrophils, eosinophils and basophils) and mononuclear leukocytes (monocytes and lymphocytes); thrombocytes (platelets); immune cells or lineage committed cells; cancerous cells; viral and retroviral cells; bacteriophage and other bacterial cells; other pathogenic cells; and so forth.

"Hybridization assay" is used herein to mean an assay that uses a hybridization reaction to measure the presence or concentration of a given polynucleotide analyte. Exemplary polynucleotide analytes include nucleic acids; genes; chromosomes; plasmids; genomes of bacteria, yeasts, viruses, viroids, molds, fungi, plants, animals, humans and fragments thereof; and so forth. The term "nucleic acid" includes DNA (including, single stranded ssDNA, double stranded dsDNA and branched bDNA), RNA (including t-RNA, m-RNA, r-RNA), mitochondrial DNA and RNA, chloroplast DNA and RNA, DNA-RNA hybrids, and mixtures thereof. Hybridization assay include, by way of illustration and not limitation, bDNA assays, polymerase chain reaction and reverse transcriptase-polymerase chain reaction, sandwich hybridization assays, nucleic acid sequence-based amplification, RNAse protection assays, sequencing, ligation assays, primer extension, and so forth.

"Immunoassay" is used herein to mean an assay that uses an immunological reaction to measure the presence or concentration of a given analyte (e.g., an antigen). Immunoassays include, by way of illustration and not limitation, solid-phase ELISA immunoassays, competitive immunoassays, sandwich assays, radioimmunoassays, fluorescence immunoassays, and so forth.

"Non-specific binding" is used herein to refer to the non-covalent binding of the magnetic particles to cells contained within a sample solution.

"Protein-containing materials" is used herein to refer to cells, immunoglobulins, proteins, enzymes, and so forth.

"Sample" is used herein to refer to a biological sample that is to be analyzed by an assay method, and therefore is suited for use in the methods of the invention. The sample is typically obtained from an individual" and thus, the specimen may be a solid tissue sample (e.g., a sample of tissue obtained from a biopsy), a fluid sample (e.g., a blood or urine sample), or any other patient specimen commonly used in the medical community, for example a cultured cell sample or fecal sample. In some embodiments of the invention, the fluid sample can be a body fluid such as lymph fluid, lysates of cells, milk, plasma, saliva, semen, serum, spinal fluid, tears, whole blood, fractions of whole blood, wound samples, the external sections of the skin, and the secretions of the respiratory, intestinal, and genitourinary tracts. Preferably, the sample is a tissue sample, whole blood sample, white blood cell sample, plasma sample, serum sample, cultured cell sample, fecal sample, or urine sample.

### SAMPLE PREPARATION METHODS

One embodiment of the invention is a method for preparing a sample for use in an assay comprising:
(a) adding a polyalkylene glycol and magnetic particles to a sample to form a mixture, wherein the magnetic particles are capable of non-specific binding to protein-containing materials contained within the mixture and wherein the mixture has a salt concentration of less than or equal to about 0.2M;
(b) precipitating the protein-containing materials out of the mixture when the protein-containing materials become non-specifically bound to the magnetic particles, to form a precipitate and a supernatant;
(c) discarding the supernatant; and
(d) isolating the precipitate to produce a prepared sample.

Numerous polyalkylene glycols can be used in the methods of the invention, and include, by way of illustration and not limitation, polyethylene glycols, polypropylene glycols and polybutylene glycols, as well as combinations thereof. Preferred polyalkylene glycols are polyethylene glycol (PEG) polymers, which are condensation products of ethylene glycol and have the general formula: HO-(CH₂CH₂-O)ₓ-H. PEG is readily available from numerous commercial sources and is typically sold as a mixture of PEG polymers of varying molecular weights. PEG having an average molecular weight within the range of about 300 to 30,000 is particularly well suited for use in the methods of the invention, more specifically 2000-15,000, with particular molecular weights of about 8000 being particularly preferred.

There are numerous magnetic particles that are suited for use in the methods and kits of the invention, and include paramagnetic particles and magnetic latex beads.

Magnetic particles or magnetic beads are commercially available from sources such as PerSeptive Biosystems, Inc. (Framingham, ME), Bayer Diagnostics (Medfield, MA), Bangs Laboratories (Carmel, IN), and BioQuest, Inc. (Atkinson NH). Exemplary materials include iron, iron oxide, iron nitride, iron carbide, nickel and cobalt, as well as mixtures and alloys thereof.

Magnetic latex beads are commercially available form source such as Seradyne.

The magnetic particles typically have an average diameter about 0.1-100 µm in diameter, preferably about 1-50 µm, more preferably about 1-5 µm. The shape of the magnetic particles will commonly be spherical, but particles of other configurations, irregular, rod-like, etc., may also be used.

While the salt concentration is preferably less than or equal to about 0.2M, it may be desirable to utilize a salt concentration of less than or equal to about 0.125M. Typically, the method will operate within acceptable parameters by having a salt concentration within the range of about 0.01-0.10M. Typical salts include, by way of illustration and not limitation, NaCl, MgCl₂, LiCl, KCI, and so forth.

The precipitating step can be done solely under gravity as the protein-containing material-magnetic particle complexes settle. It may be desired to facilitate the speed of this process by application of a magnetic field to the bottom of the reaction vessel. Thus, the precipitate can be isolated by allowing the magnetic particle complexes to settle under gravity or with application of a magnetic field at some point during the settling stage. In another embodiment, a magnetic filed is applied while the supernatent is being discarded in order to maximize the amount of isolated precipitate.

The prepared sample can then be used immediately in an assay or stored for subsequent use, under appropriate storage conditions.

When the prepared sample is to be stored for subsequent use, no further processing is needed. After the supernatant is discarded and the precipitate is isolated to produce the prepared sample, the sample is frozen. Typical storage conditions are at temperatures within the range of +4 to -80°C. Preferred storage condition are at a temperature within the range of -20 to -80°C, for up to six months or longer.

When the prepared sample is to be used immediately in an assay, it may be desirable to further process the sample, typically to separate the protein-containing materials from the magnetic particles. Accordingly another embodiment of the method of the invention further comprises:
(e) dissolving the prepared sample in a solution to form a mixture of unbound protein-containing materials and magnetic particles;
(f) applying a magnetic field to the mixture to remove the magnetic particles;
(g) collecting the protein-containing materials to form an isolated prepared sample.
The solution used in this dissolving step can either be a standard buffer solution or a lysis solution. Selection of the type of solution will depend upon whether it is desirable to maintain the integrity of any cellular matter in the protein-containing materials (e.g. for use in an immunoassay), or whether it is desirable to disrupt the cells to release any DNA or RNA contained therein (e.g., for use in a hybridization assay).

The method may also involve the addition of a detergent, which may be cationic, anionic, ionic, etc. Cationic detergents are preferred and exemplary cationic detergents include, by way of illustration and not limitation, quaternary ammonium salts such as cetyl trimethyl ammonium bromide and octadecyl trimethyl ammonium salt. The detergent can be present at a concentration the range of about 1-10 mM.

Since viruses are known to be associated with various proteins within samples, the inclusion of a detergent in the precipitation step facilitates a more efficient release of virus (and subsequent nucleic acids) from these bound proteins making them accessible in the bDNA hybridization assay. Inclusion of detergent solely in the bDNA assay did not generate higher signals than the control suggesting that this detergent is not accelerating the hybridization reaction (Pontius and Berg, 1991). In addition, a 16-18 hour hybridization reaction is used in the bDNA assay protocol thus eliminating any potential hybridization acceleration.

In a preferred embodiment of the invention, the sample is a blood sample and the method for preparing a blood sample for use in an assay comprises:
(a) adding a polyalkylene glycol and magnetic particles to a blood sample to form a mixture, wherein the magnetic particles are capables of non-specific binding to cells contained within the mixture and wherein the mixture has a salt concentration of less than or equal to about 0.2M;
(b) precipitating the cells out of the mixture when the cells become non-specifically bound to the magnetic particles, to form a precipitate and a supernatant;
(c) discarding the supernatant; and
(d) isolating the precipitate to produce a prepared blood sample.
The prepared blood sample can be stored or further treated as described above, for immediate use in an assay.

### IMMUNOASSAYS

The invention finds particular utility as a sample preparation method for immunoassays. In those instances, the analyte is typically a protein. Any art-known immunoassay that can detect proteins may be used.

In general, immunoassays involve techniques that make use of the specific binding between an epitope on a molecule and its homologous antibody in order to identify and preferably quantify a substance in a sample. Thus, the immunoassays used to measure protein markers make use of specific binding between the protein marker and a corresponding antibody directed against the protein marker. One method for detecting protein markers involves placing the sample on a slide, adding an appropriately labeled antibody, washing unbound labeled antibody, and viewing the sample with an appropriate device, e.g., microscope, for the presence of bound protein.

Another type of immunoassay involves solid-bound antibodies directed against a particular protein marker are contacted with the patient specimen in order to immobilize the particular protein marker. After unbound protein is washed, a second labeled antibody directed to a different epitope on the protein marker is contacted with the immobilized protein. The labeled antibody is detected and quantified. Specific immunoassays are well known to those of ordinary skill in the art. For example, enzyme immunoassays such as an enzyme-linked immunosorbant assay (ELISA) employ an enzyme as the detectable label.

Antibodies specific for the protein analyte may be available commercially or produced using art-known methods such as monoclonal or polyclonal production of antibodies. For example, a protein is injected into a host (e.g., rabbit or mouse), and its spleen is removed several weeks later. In the presence of ethylene glycol, spleen cells from the host are added to myeloma cells that lack hypoxanthine-guanosine phosphotibosyl transferase (HGPRT). In a medium that contains hypoxanthine, aminopterin and thymine, only fused cells survive since the unfused spleen cells do not grow *in vitro* and unfused myeloma cells cannot create new nucleotides in the medium without HGPRT. The fused cells can then be tested for the production of the desired antibody and subsequently separated and cultured. The result is a supply of antibodies directed against the protein. Depending on the assay design, the antibodies may be labeled prior to use.

### HYBRIDIZATION ASSAYS

The invention also finds utility as a sample preparation method for hybridization assays such as branched chain DNA (bDNA) assays and reverse transcriptase-polymerase chain reaction (RT-PCR) assays. Such assays are well known in the art and are typically conducted using commercially available kits. For example, an RT-PCR test to detect the presence of HCV RNA is the Amplicor HCV Monitor test (Roche Diagnostics, Molecular Systems Division, Nutley, NJ), while a bDNA test for HCV RNA is the Quantiplex HCV-RNA 2.0 test (Bayer Diagnostics).

Nucleic acid hybridization is a well known method for identifying specific sequences of nucleic acids or polynucleotides. In general, hybridization is based upon pairing between complementary nucleic acid strands. Single-stranded oligonucleotides having known sequences can be used as probes to identify target sequences of nucleic acid analytes, by exposing the probes to sample solutions containing nucleic acid analytes of interest. If a nucleic acid analyte hybridizes to a probe, the analyte necessarily contains the target sequence. Various aspects of this method have been studied in detail. In essence, all variations of hybridization assays allow complementary base sequences to pair and form double-stranded molecules and it is understood that the methods of the invention can be used in all types of hybridization assays. Details of some of these assays are provided below.

### Sandwich Hybridization Assays

Sandwich hybridization assays are well known in the art. See, for example, U.S. Patent Nos. 5,124,246, 5,710,264 and 5,849,481 to Urdea et al. Briefly, a sample such as a patient specimen is placed in contact with oligonucleotide probes under hybridizing conditions. The oligonucleotide probes then hybridize to any mRNA of interest that is present in the sample. The probes are typically immobilized on a substrate so as to capture or immobilize complementary mRNA. The sample remains in contact with the substrate-bound oligonucleotide probes for a period of time sufficient to ensure that hybridization to the oligonucleotide probes is complete. One skilled in the art can determine necessary "incubation" times, but a time of from about 0.25 hours to about 3.0 hours is typical. After a sufficient incubation time has elapsed, the sample is washed with a suitable washing solution so as to remove unhybridized material. Washing techniques are well-known and/or can be readily determined by one of ordinary skill in the art. Typically, a washing fluid is employed that comprises a buffer solution and possibly a detergent. The buffer solution may be any conventional solution known in the art suitable for removing unhybridized material, and commonly contains one or more salts of alkali metals such as sodium chloride and sodium citrate or combinations thereof. The detergent may be any detergent that is suitable for washing unbound oligonucleotide probes, such as the non-ionic polyoxyethylene-based detergents sold under the brand names Brij^{®} , Triton^{®} , Tween^{®} , Genapol^{®} , Igepal Ca^{®} , Thesit^{®} , and Lubrol^{®}. All of these are commercially available from commercial suppliers such as Sigma Corp. (St. Louis, MO. One or more washing are done at temperatures within the range of about 21-60°C. Optimally, the wash step is carried out at room temperature.

### Reverse Transcriptase-Polymerase Chain Reaction

Reverse transcriptase-polymerase chain reaction (RT-PCR) hybridization assays involve making cDNA based on any mRNA present in a sample, followed by measurement. Such techniques are well known in the art. Generally, an excess of the four deoxynucleotide triphosphate molecules (i.e., deoxyadenosine triphosphate, deoxycytidine triphosphate, deoxythymidine triphosphate and deoxyguanosine triphosphate), and a primer, i.e., an oligo-dT primer, are added to the sample. After separation from the mRNA strand (by denaturing in a basic medium, for example), the resulting oligonucleotide is a single-stranded DNA complementary to the original mRNA sequence. A DNA polymerase may then be added in the presence of an excess of the four deoxynucleotide triphosphate molecules and a primer to create double-stranded DNA. Further details are provided in Gerard et al., *Mol. Biotechnol.* 8(1):61-77 (1997) and Ando et al., *J. Clin Microbiol.* 35(3):570-577 (1997). Thereafter, the double-stranded DNA can be denatured into single-stranded DNA wherein one of the two strands is essentially a DNA corresponding to the original mRNA. The DNA, however, is less susceptible to degradation and may therefore be used as a more stable surrogate for mRNA in determining the amount of the mRNA in a sample. A variety of methods to detect DNA are known and may be used to detect and determine the amount of the corresponding mRNA originally contained in the sample. As will be appreciated, many of the methods for detecting and measuring mRNA described herein can be adapted to detect and measure DNA.

### Transcription-Mediated Amplification

Transcription-mediated amplification (TMA) assays are similar to RT-PCR assays in that reverse transcriptase is added to a sample to create cDNA of the target mRNA. However, in TMA, a RNA polymerase is added to synthesize RNA amplicons using cDNA as a template. Each of the newly synthesized amplicons reenters the TMA process and serves as a template for a new round of replication. Thus, the TMA process results in the effective amplification of the mRNA. The RNA amplicons are then detected and measured by labeled probes complementary for the RNA amplicons. Similar TMA-based assays are described in the literature. See, for example, Pasternack et al. *J. Clin. Microbiol.* 35(3):676-678 (1997).

### Nucleic Acid Sequence-Based Amplification

Nucleic acid sequence-based amplification or (NASBA^{®}) is a homogenous amplification process. The method involves the addition of three enzymes (reverse transcriptase, RNase H, and T7 RNA polymerase) and two primers to a single reaction vessel containing mRNA from the sample. The first primer contains a 3' terminal sequence that is complementary to a sequence on the mRNA and a 5' terminal sequence that is recognized by the T7 RNA polymerase. In combination, these reagents result in the synthesis of multiple copies of mRNA that can then be measured by adding an appropriate labeled probe. This type of assay is well-known in the art and is described in, for example, Davey et al. EP 0329822.

### RNAse Protection Assay

In RNAse protection assays, a labeled oligonucleotide probe is added to the sample resulting in the hybridization between the labeled probe and any complementary mRNA. The sample is then treated with RNase to degrade all remaining single-stranded mRNA. Hybridized portions of the probe will be protected from digestion. Unhybridized fragments can be separated from the larger, hybridized complexes that bear a label by, for example, electrophoresis. The label can then be measured. If the probe is added at a molar excess, e.g., at least twice molar excess, with respect to the mRNA, the resulting signal is proportional to the amount of mRNA in the sample.

### Branched DNA Assays

Branched DNA (bDNA) are described in detail in Urdea et al., Gene 61:253-264 (1987). Other exemplary bDNA assays are described in US Patent No. 4,868,105 to Urdea, et al.; US Patent No. 5,124,246 to Urdea et al.; US Patent No. 5,132,204 to Urdea et al.; US Patent No. 5,635,352 to Urdea et al; and US Patent No. 5,681,702 to Collins et al. Example 3 illustrates one such protocol.

### KITS

Another embodiment of the invention is a kit useful in preparing samples for use in an assay comprising:
(a) a polyalkylene glycol;
(b) magnetic particles that are capable of non-specific binding to protein-containing materials contained within the samples;
(c) salt having a concentration of less than or equal to about 0.2M; and
(d) instructions for preparing the samples.
Other materials useful in the performance of the assays can also be included in the kits, including test tubes, magnets, transfer pipettes, and the like. Kits can contain reagents, nucleic acid probes, labeled analyte, and standards. Kits can contain materials sufficient for one assay, or can contain sufficient materials for multiple assays, for example, materials for 10, 25, 50, or more assays can be provided in a single kit. The kits can also include instructions for the use of one or more of these reagents in any of the assays described herein.

### EXAMPLES

The practice of the present invention will employ, unless otherwise indicated, conventional techniques of pharmaceutical formulation, medicinal chemistry, and the like, which are within the skill of the art. Such techniques are explained fully in the literature. Preparation of various types of pharmaceutical formulations are described, for example, in *Remington: The Science and Practice of Pharmacy,* Nineteenth Edition. (1995) cited *supra* and Ansel et al., *Pharmaceutical Dosage Forms and Drug Delivery Systems,* 6^{th} Ed. (Media, PA: Williams & Wilkins, 1995).

The following examples are put forth so as to provide those of ordinary skill in the art with a complete disclosure and description of how to make and use the compounds of the invention, and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C and pressure is at or near atmospheric. All components were obtained commercially unless otherwise indicated.

### ABBREVIATIONS

- bDNA: branched chain DNA
- BGG: bovine gamma globulin
- DS: dextran sulfate
- HBV: hepatitis B virus
- HCV: hepatitis C virus
- HIV: human immunodeficiency virus
- Meq: million equivalents
- MLP: magnetic latex particles
- PBMC: peripheral blood mononuclear cell
- PBS: phosphate-buffered saline
- PBS/Tx-100: 1X Dulbecco's Solution, 0.1% Tx-100
- PEG: polyethylene glycol
- PMP: paramagnetic particles
- SA: Streptavidin

### EXAMPLE 1

### Increased adsorption of PEG-precipitated proteins to magnetic particles in low salt medium

### A. BGG Separation-PEG/NaCl

A solution of heat-aggregated BGG in PBS was prepared by heating the BGG at 60°C for 20 minutes. The solution had initial optical density (280 nm) of 0.362, and became highly turbid when 0.15 ml of 17.2% PEG (vol/vol) plus 3M NaCl was added to 0.5 ml protein solution. The turbid mixture was then cooled for 30 min on ice and spun for 20 minutes at 2000 rpm to sediment the precipitated protein. This process completely clarified the solution and the optical density was reduced to 0.03, indicating removal of most of the BGG from the solution.

### B. BGG Separation-PEG/NaCl and PMP

The BGG separation experiment was repeated, except that after the PEG/NaCl addition, 1 mg PMP was added, followed by magnetic separation. There was only a small reduction in turbidity observed and no significant change in optical density.

### C. BGG Separation-PEG and PMP

The BGG separation experiment was repeated, except that NaCl was omitted from the PEG solution. The same degree of turbidity developed initially, and when 1 mg PMP was added, a magnetic separation completely clarified the solution. This indicated that, in the absence of salt, the precipitated protein adhered to PMP more efficiently.

A similar experiment was done using 20 different human sera instead of heat-aggregated BGG. In all of these sera, clarification of PEG-induced turbidity was more efficient in the absence of the NaCl. Clarification was achieved using PMP or MLP (Seradyne).

Subsequent experiments bDNA assays for HCV and HIV indicated that-sensitivity was 3-5 times higher with PEG/PMP mixtures containing 0.01-0.10M NaCl as compared to 1M NaCl.

### EXAMPLE 2

### A. Hepatitis B bDNA Hybridization Assay: Evaluation of Viral Concentration Using PEG/PMP with and without MgCl₂ and Dextran Sulfate, Release with PBS/Tx-100 and Lysis Solution

### i. Capture and Release Protocol

A 500 µl serum sample was mixed with 150 µl of a PEG/PMP solution (1mg PMP, 17.2% PEG (vol/vol)). The mixture was vortexed and the precipitate separated. 100 µl of supernatant was removed and retained for unbound determination. 1 0 µl of supernatant was transferred to a plate when the released virus was transferred to the plate. The remaining supernatant was removed and discarded.

150 µl of lysis solution or PBS/Tx-100 was added to the precipitate, vortexed and incubated at 63°C for 30 minutes. The PMP were separated out. 10 µl of the released virus was transferred to the plate.

### ii. Quantitation

The bDNA assay was then run per the manufacturer's protocol. The HBV kit used was MM11225 (Bayer Diagnostics). Positive serum input was 375 Meq/sample. Buffer A was PBS/Tx-100. Buffer B was a lysis solution.

**Table 1: Unbound**

| | |
|---|---|
| 17.2% PEG (vol/vol) | 98 Meq/sample |
| 17.2% PEG, 0.09% DS | 81 Meq/sample |
| 17.2% PEG, 2% DS | 20 Meq/sample |
| 17.2% PEG, 0.09% DS, 90mM | 72 Meq/sample |
| MgCl₂ | |

**Table 2: Released**

| Buffer | Precipitating Agent | Meq released | % released | Meq unbound | Total recovered (rel + unb) | % recovered |
|---|---|---|---|---|---|---|
| A | 17.2%PEG | 203 | 54 | 98 | 300 | 84 |
| A | 17.2% PEG | 173 | 46 | 81 | 254 | 68 |
| | and 0.09% | | | | | |
| | DS | | | | | |
| A | 17.2% PEG | 263 | 70 | 20 | 282 | 75 |
| | and 2% DS | | | | | |
| A | 172% PEG, | 233 | 62 | 72 | 304 | 81 |
| | 0.09% DS, | | | | | |
| | and 90 mM | | | | | |
| | MgCl₂ | | | | | |
| B | 17.2% PEG | 184 | 49 | 98 | 281 | 75 |
| B | 17.2% PEG | 184 | 49 | 81 | 265 | 71 |
| | and 0.09% | | | | | |
| | DS | | | | | |
| B | 17.2% PEG | 323 | 86 | 20 | 342 | 91 |
| | and 2% DS | | | | | |
| B | 17.2% PEG, | 300 | 80 | 72 | 372 | 99 |
| | 0.09% DS, | | | | | |
| | and 90 mM | | | | | |
| | MgCl₂ | | | | | |

### B. Hepatitis B bDNA Hybridization Assay with Concentration Step: Evaluation of 4 Concentrations of Dextran Sulfate in PEG/PMP Precipitating Reagent and Effect of pH of PBS/Tris/Tx-100 Releasing Agent

The PBS/Tris composition used was 50 mM sodium phosphate, 50 mM Tris, 150 mM NaCl, 1 mM ethylenediaminetetraacetic acid, tetrasodium salt, and 0.02%NaN₃.

**Table 3: PEG and DS Concentrations (% vol/vol)**

| | | Composition | | | |
|---|---|---|---|---|---|
| | | A | B | C | D |
| In the | PEG | 17.2 | 17.2 | 17.2 | 17.2 |
| working reagent | DS | 0 | 2 | 4 | 8 |
| In the | PEG | 4.0 | 4.0 | 4.0 | 4.0 |
| Reaction* (500 µl serum + 150 µl reagent) | DS | 0 | 0.5 | 0.9 | 1.8 |

| | | | | | |
|---|---|---|---|---|---|
| * 1 mg of NH₂-PMP/500µl of serum was also present | | | | | |

**Table 4: Dextran Sulfate Input (%)**

| | Composition | | | |
|---|---|---|---|---|
| | A | B | C | D |
| PMP Pretreated with PBS: | 0 | 0.5 | 0.9 | 1.8 |

| Releasing agent/buffer pH: | | | | |
|---|---|---|---|---|
| bDNA Lysis Solution (pH 8) | | | | |
| milli-equivalents | 81 | 78 | 63 | 66 |
| % released | 73 | 71 | 57 | 60 |

| PBS/Tris/0.1% Tx-100 (pH 7.0) | | | | |
|---|---|---|---|---|
| milli-equivalents | 83 | 95 | 70 | 67 |
| % released | 75 | 86 | 64 | 60 |

| PBS/Tris/0.1% Tx-100 (pH 9.0) | | | | |
|---|---|---|---|---|
| milli-equivalents | 73 | 92 | 64 | 71 |
| % released | 66 | 83 | 76 | 65 |
| Input: milli-equivalents | 101 | 110 | 110 | 110 |

### EXAMPLE 3

### Hepatitis C bDNA Hybridization Assay

### A. Capture of HCV using NH₂-MLP, NH₂-PMP and BGG-PMP/PEG Precipitation Protocol

The following three solid phases (50 mg/ml stocks) were used: NH₂-MLP (prepared by coupling of bis-amine to COOH MLP (Seradyne)), BGG-PMP and NH₂-PMP.

**Table 5: PEG/PMP or MLP Precipitation Protocol**

| | No PMP control | No PMP + PEG control | NH₂-MLP | NH₂-MLP +PEG |
|---|---|---|---|---|
| Sample | 500 µl | 500 µl | 500 µl | 500 µl |
| Water | 10 µl | 10 µl | 0 µl | 0 µl |
| Solid Phase | 0 µl | 0 µl | 10 µl | 10 µl |
| (1 mg) | | | | |
| | | vortex and add | | |
| Water | 170 µl | 0 µl | 170 µl | 0 µl |
| PEG | 0 µl | 170 µl | 0 µl | 170 µl |
| (17.2%) | | | | |

| | NH₂-PMP | NH₂-PMP + PEG | BGG-PMP | BGG-PMP+ PEG |
|---|---|---|---|---|
| Sample | 500 µl | 500 µl | 500 µl | 500 µl |
| Water | 0 µl | 0 µl | 0 µl | 0 µl |
| Solid | 10 µl | 10 µl | 10 µl | 10 µl |
| Phase (1 | | | | |
| mg) | | | | |
| | | vortex and add | | |
| Water | 170 µl | 0 µl | 170 µl | 0 µl |
| PEG | 0 µl | 170 µl | 0 µl | 170 µl |
| (17.2%) | | | | |

The serum became cloudy upon addition of PEG as the protein precipitated out of solution. The precipitate (solid phase) was removed. 50 µl neat and 50 µl of 10X dilution (25 µl + 225 µl negative serum) was transferred to the bDNA plate. The remainder of the supernatant was removed and discarded.

For samples without solid phase transfer, 125 µl of the sample plus 375 µl of the lysis solution was to placed in an Eppendorf tube and incubate at 53°C for 1 hour. 150 µl was then transferred to the plate.

500 µl of lysis reagent was added to the solid phases and then transferred to Eppendorf tubes and incubated at 53°C for 60 minutes in an Eppendorf microfuge. The solid phase was then separated out. 150 µl neat and 150 µl of 10X dilution (50 µl + 400 µl lysis solution) was transferred to the bDNA plate. The bDNA assay was then run per the manufacturer's protocol. The HCV kit used was the HCV 2.0 bDNA kit, MM833 (Bayer Diagnostics).

**Table 6: RNA Remaining in Serum Following Treatment**

| | | Without PEG | With PEG |
|---|---|---|---|
| Positive serum | Meq/sample | 64.0 | 74.1 |
| NH₂-MLP | Meq | 74.1 | 7.0 |
| | % of input (unbound) | 100.0 | 9.4 |
| | bound Meq | 0.0 | 67.1 |
| NH₂-PMP | Meq | 67.0 | 6.9 |
| | % of input (unbound) | 100.0 | 9.3 |
| | bound Meq | 0.0 | 67.1 |
| BGG-PMP | Meq | 66.8 | 14.3 |
| | % of input (unbound) | 100.0 | 19.3 |
| | bound Meq | 0.0 | 59.8 |

**Table 7: RNA Released from Solid Phase Surface**

| | | Without PEGMeq/sample | With PEG Meq/sample |
|---|---|---|---|
| Positive serum | Meq/sample | 116.8 | 118.6 |
| NH₂-MLP | Meq | 2.5 | 116.5 |
| | % released | 2.1 | 98.2 |
| NH₂-PMP | Meq | 8.3 | 85.0 |
| | % released | 7.1 | 71.7 |
| BGG-PMP | Meq | 5.4 | 101.8 |
| | % released | 5.7 | 85.9 |

### B. Capture of HCV using Anti Envelope and Anti Core PMP or a PEG/PMP Precipitation

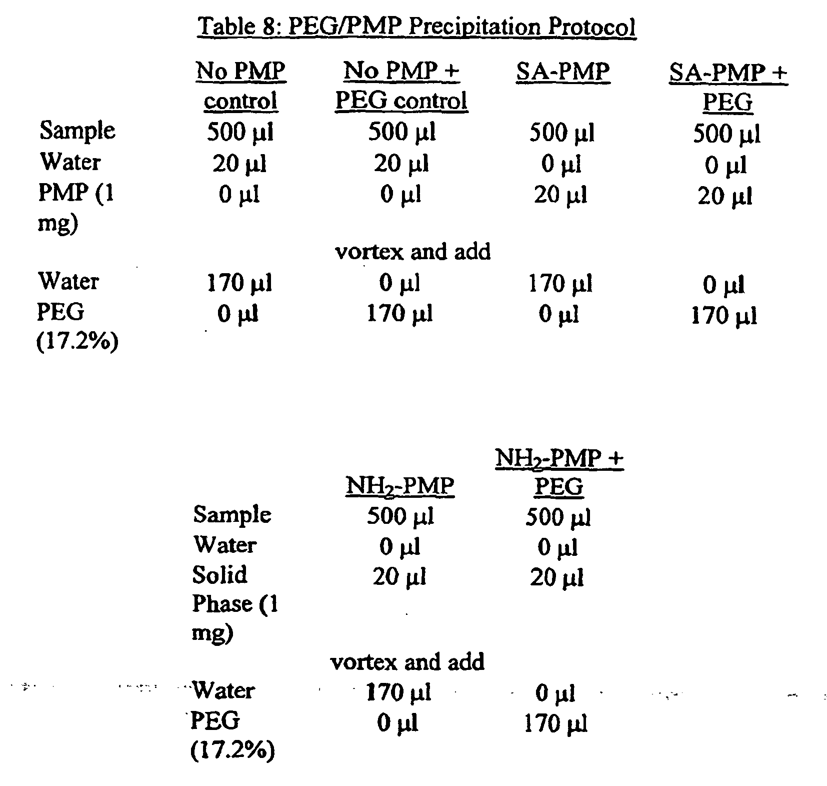

The serum became cloudy upon addition of PEG as the protein precipitated out of solution. The precipitate (solid phase) was removed. 50 µl neat and 50 µl of 10X dilution (25 µl + 225 µl negative serum) was transferred to the bDNA plate. The bDNA assay was then run per the manufacturer's protocol.

**Table 9: RNA Remaining in Serum Following Treatment**

| | | Without PEG Meq | With PEG Meq |
|---|---|---|---|
| Input (no PMP) | | 129.1 | 129 |
| + 1 mg streptavidin PMP | | 128.5 | 6.1 |
| | % of input (unbound) | 99.5 | 4.7 |
| + 1 mg NH₂-PMP | | 93.2 | 5.3 |
| without Immobilized | | | |
| Protein | | | |
| | % of input (unbound) | 72.2 | 4.1 |

### EXAMPLE 4

### Cell Assays

The same procedures described above were applied to cultured cells (Jurkat) and white blood cells, and the number of cells captured was counted by microscopy. Both types of cells were captured at more than 90% yield.

### A. Purification of PBMC's from 1 ml Whole Blood: Materials/Methods

Materials: 1 ml of whole blood; 300 µl PMP/PEG (3.33mg PMP/8.75% PEG); Milli-Q Water, 10 x PBS and 1 x PBS; and whole blood on ice.

5 ml of whole blood was resuspended in 45 mls of Milli-Q water, and inverted for approximately 30 seconds. 5 ml of 10 x PBS was added. The mixture was spun at 1000 g for 10 minutes and the supernatant was aspirated out. The wash was repeated with 50 mls 1 x PBS, and then resuspended in 5 ml of 1 x PBS to provide the cell suspension. A control aliquot was made by placing 1 ml of the cell suspension into 1.5 Eppendorf tubes (hold on ice). 1 ml of the cell suspension was added to 300 µl of the PMP/PEG reagent, and the mixture inverted 5 times. The mixture was then placed on magnets and separated for 5 minutes. The supernatant was removed and placed in 1.5 ml Eppendorf tubes. The control and sample Eppendorf tubes were spun for 10 minutes at 2000 g in and Eppendorf centrifuge. The liquid was aspirated and the pellet resuspended in 100 µl of PBS.

### B. Evaluation of Method: Viable cell counts

10µl of Trypan Blue (4%) was placed in an Eppendorf tube. 10 µl of the resuspended cells were added. The number of viable cells was counted in a hemocytometer (4 grids or 200 cells). The control (diluted 1:100 in PBS), 10 (x 100)cells/4 grids x 1.0e4 had a viable cell count of 2,500,000 cells/ml. No viable cells were counted in the PMP/PEG supernatant. Thus, the PMP/PEG treatment of the invention was successful in removing all cells from the sample.

### EXAMPLE 4

### Detection of Glyceraldehyde-3-phosphate dehydrogenase (GAPDH) from hypotonically lysed PBMC's with PMP/PEG

A bDNA assay for GAPDH, a housekeeping gene found in cells, was used to monitor the linearity of PMP/PEG sample concentration. One ml. of hypotonically prepared peripheral blood mononuclear cells (PBMCs) (5.5e4 cells/ml) or whole blood (WB) was diluted in 1x PBS to the ratios shown below, and concentrated by the PMP/PEG method as described. The resulting concentrated sample was analyzed in a bDNA assay for GAPDH. As shown below, the Signal/Noise (S/N) ratios are linear down to the lowest dilution tested with both PBMC's and Whole Blood supporting the broad applicability of this sample preparation method with whole blood, cultured cells, etc.

**Table 10: GAPDH bDNA S/N Values From PMP/PEG Concentrated PBMC's and Whole Blood**

| PBMCs | 1x PBS | cells/well | S/N | WB | 1x PBS | S/N |
|---|---|---|---|---|---|---|
| 1 | 0 | 55600 | 48.8 | 1 | 0 | 63.7 |
| 0.75 | 0.25 | 41700 | 34.2 | 0.75 | 0.25 | 48.2 |
| 0.5 | 0.50 | 20850 | 21.7 | 0.5 | 0.50 | 51.3 |
| 0.25 | 0.75 | 10425 | 10.1 | 0.25 | 0.75 | 35.4 |
| 0.125 | 0.875 | 5213 | 5.1 | 0.125 | 0.875 | 25.7 |

## Claims

1. A method for preparing a sample for use in an assay comprising:
(a) adding a polyalkylene glycol and magnetic particles to a sample to form a mixture, wherein the magnetic particles are capable of non-specific binding to protein-containing materials contained within the mixture and wherein the mixture has a salt concentration of less than or equal to about 0.2M;
(b) precipitating the protein-containing materials out of the mixture when the protein-containing materials become non-specifically bound to the magnetic particles, to form a precipitate and a supernatant;
(c) discarding the supernatant; and
(d) isolating the precipitate to produce a prepared sample.

2. The method of Claim 1 which further comprises:
(e) dissolving the prepared sample in a solution to form a mixture of unbound protein-containing materials and magnetic particles;
(f) applying a magnetic field to the mixture to remove the magnetic particles;
(g) collecting the protein-containing materials to form an isolated prepared sample.

3. The method of claim 1 or 2 wherein the sample is a tissue sample, whole blood sample, white blood cell sample, plasma sample, serum sample, cultured cell sample, fecal sample, or urine sample.

4. A method for preparing a blood sample for use in an assay comprising:
(a) adding a polyalkylene glycol and magnetic particles to a blood sample to form a mixture, wherein the magnetic particles are capable of non-specific binding to cells contained within the mixture and wherein the mixture has a salt concentration of less than or equal to about 0.2M;
(b) precipitating the cells out of the mixture when the cells become non-specifically bound to the magnetic particles, to form a precipitate and a supernatant;
(c) discarding the supernatant; and
(d) isolating the precipitate to produce a prepared blood sample.

5. The method of claim 4 which further comprises:
(e) dissolving the prepared blood sample in a solution to form a mixture of unbound blood cells and magnetic particles;
(f) applying a magnetic field to the mixture to remove the magnetic particles;
(g) collecting the blood cells to form an isolated prepared blood sample.

6. The method of any preceding claim wherein the precipitating step further comprises application of a magnetic field.

7. The method of any preceding claim wherein the polyalkylene glycol is polyethylene glycol.

8. The method of any preceding claim wherein the solution is a buffer solution.

9. The method of any preceding claim wherein the solution is a lysis solution.

10. The method of any preceding claim wherein the mixture has a salt concentration of less than or equal to about 0.125M.

11. The method of claim 10 wherein the mixture has a salt concentration within the range of about 0.01-0.10M.

12. The method of any preceding claim wherein the salt is selected from the group consisting of NaCl, MgCl₂, LiCl and KCl.

13. The method of any preceding claim wherein the mixture further comprises a detergent.

14. The method of any preceding claim wherein the detergent is present at a concentration of about 1-10 mM.

15. The method of claim 13 or 14 wherein the detergent is a cationic detergent.

16. The method of claim 15 wherein the cationic detergent is a quaternary ammonium salt.

17. The method of claim 16 wherein the quaternary ammonium salt is selected from the group consisting of cetyl trimethyl ammonium bromide and octadecyl trimethyl ammonium salt.

18. The method of any preceding claim wherein the magnetic particles are comprised of a material selected from the group consisting of iron, iron oxide, iron nitride, iron carbide, nickel and cobalt, and mixtures and alloys thereof.

19. The method of any preceding claim wherein the magnetic particles are paramagnetic particles, or magnetic latex particles.

20. The method of any preceding claim wherein the magnetic particles have an average diameter within the range of about 0.1-100 µm.

21. The method of claim 20 wherein the magnetic particles have an average diameter within the range of about 1-50 µm.

22. The method of claim 21 wherein the magnetic particles have an average diameter within the range of about 1-5 µm.

23. The method of claim 1 and any claim dependent thereon wherein the protein-containing materials are selected from the group consisting of cells, immunoglobulins, proteins and enzymes.

24. The method of any preceding claim wherein the prepared sample is suitable for use in a hybridization assay.

25. The method of any preceding claim wherein the magnetic particles are coated.

26. The method of any preceding claim wherein the prepared sample is suitable for use in an immunoassay.

27. A kit useful in preparing samples for use in an assay comprising:
(a) a polyalkylene glycol;
(b) magnetic particles that are capable of non-specific binding to protein-containing materials contained within the samples;
(c) salt having a concentration of less than or equal to about 0.2M; and
(d) instructions for preparing the samples.

28. The kit of claim 27 wherein the polyalkylene glycol is polyethylene glycol.

29. The kit of claim 27 or 28 which further comprises a buffer solution.

30. The kit of claim 27 or 28 which further comprises a lysis solution.

31. The kit of any one of claims 27-30 wherein the salt has a concentration of less than or equal to about 0.125M.

32. The kit of claim 31 wherein the salt has a concentration within the range of about 0.01-0.10M.

33. The kit of any one of claims 27-32 wherein the salt is selected from the group consisting of NaCl, MgCl₂, LiCl and KCl.

34. The kit of any one of claims 27-33 which further comprises a detergent.

35. The kit of any one of claims 27-34 wherein the detergent is a cationic detergent.

36. The kit of claim 35 wherein the cationic detergent is a quaternary ammonium salt.

37. The kit of claim 36 wherein the quaternary ammonium salt is selected from the group consisting of cetyl trimethyl ammonium bromide and octadecyl trimethyl ammonium salt.

38. The kit of any one of claims 27-37 wherein the magnetic particles are comprised of a material selected from the group consisting of iron, iron oxide, iron nitride, iron carbide, nickel and cobalt, and mixtures and alloys thereof.

39. The kit of any one of claims 27-38 wherein the magnetic particles are paramagnetic particles, or magnetic latex particles.

40. The kit of any one of claims 27-39 wherein the magnetic particles have an average diameter within the range of about 0.1-100 µm.

## Patentansprüche

1. Verfahren zur Herstellung einer Probe für die Verwendung in einem Assay, welches folgendes umfaßt:
(a) Zugeben eines Polyalkylenglycols und magnetischer Teilchen zu einer Probe unter Bildung eines Gemischs, wobei die magnetischen Teilchen in der Lage sind, unspezifisch an Protein enthaltende Materialien, die in dem Gemisch enthalten sind, zu binden, und wobei das Gemisch eine Salzkonzentration von weniger als oder gleich etwa 0,2 M hat,
(b) Präzipitieren der Protein enthaltenden Materialien aus dem Gemisch, wenn die Protein enthaltenden Materialien unspezifisch an die magnetischen Teilchen gebunden werden, unter Bildung eines Präzipitats und eines Überstands,
(c) Verwerfen des Überstands und
(d) Isolieren des Präzipitats unter Erzeugung einer hergestellten Probe.

2. Verfahren nach Anspruch 1, welches weiterhin folgendes umfaßt:
(e) Lösen der hergestellten Probe in einer Lösung unter Bildung eines Gemischs von ungebundenen Protein enthaltenden Materialien und magnetischen Teilchen,
(f) Anlegen eines magnetischen Feldes auf das Gemisch unter Entfernung der magnetischen Teilchen,
(g) Sammeln der Protein enthaltenden Materialien unter Bildung einer isolierten hergestellten Probe.

3. Verfahren nach Anspruch 1 oder 2, wobei die Probe eine Gewebeprobe, eine Gesamtblutprobe, eine Probe weißer Blutkörperchen, eine Plasmaprobe, eine Serumprobe, eine Probe kultivierter Zellen, eine Fäkalienprobe oder eine Urinprobe ist.

4. Verfahren zur Herstellung einer Blutprobe für die Verwendung in einem Assay, welches folgendes umfaßt:
(a) Zugeben eines Polyalkylenglycols und magnetischer Teilchen zu einer Blutprobe unter Bildung eines Gemischs, wobei die magnetischen Teilchen in der Lage sind, unspezifisch an Zellen, die in dem Gemisch enthalten sind, zu binden, und wobei das Gemisch eine Salzkonzentration von weniger als oder gleich etwa 0,2 M hat,
(b) Präzipitieren der Zellen aus dem Gemisch, wenn die Zellen unspezifisch an die magnetischen Teilchen gebunden werden, unter Bildung eines Präzipitats und eines Überstands,
(c) Verwerfen des Überstands und
(d) Isolieren des Präzipitats unter Erzeugung einer hergestellten Blutprobe.

5. Verfahren nach Anspruch 4, welches weiterhin folgendes umfaßt:
(e) Lösen der hergestellten Blutprobe in einer Lösung unter Bildung eines Gemischs ungebundener Blutzellen und magnetischer Teilchen,
(f) Anlegen eines magnetischen Feldes auf das Gemisch unter Entfernung der magnetischen Teilchen,
(g) Sammeln der Blutzellen unter Bildung einer isolierten hergestellten Blutprobe.

6. Verfahren nach einem der vorangegangenen Ansprüche, wobei die Stufe des Präzipitierens weiterhin das Anlegen eines magnetischen Feldes umfaßt.

7. Verfahren nach einem der vorangegangenen Ansprüche, wobei der Polyalkylenglycol Polyethylenglycol ist.

8. Verfahren nach einem der vorangegangenen Ansprüche, wobei die Lösung eine Pufferlösung ist.

9. Verfahren nach einem der vorangegangenen Ansprüche, wobei die Lösung eine Lyselösung ist.

10. Verfahren nach einem der vorangegangenen Ansprüche, wobei das Gemisch eine Salzkonzentration von weniger als oder gleich etwa 0,125 M hat.

11. Verfahren nach Anspruch 10, wobei das Gemisch eine Salzkonzentration im Bereich von etwa 0,01-0,10 M hat.

12. Verfahren nach einem der vorangegangenen Ansprüche, wobei das Salz aus der Gruppe ausgewählt ist, bestehend aus NaCl, MgCl₂, LiCl und KCI.

13. Verfahren nach einem der vorangegangenen Ansprüche, wobei das Gemisch weiterhin ein Detergens umfaßt.

14. Verfahren nach einem der vorangegangenen Ansprüche, wobei das Detergens in einer Konzentration von etwa 1-10 mM vorliegt.

15. Verfahren nach Anspruch 13 oder 14, wobei das Detergens ein kationisches Detergens ist.

16. Verfahren nach Anspruch 15, wobei das kationische Detergens ein quaternäres Ammoniumsalz ist.

17. Verfahren nach Anspruch 16, wobei das quaternäre Ammoniumsalz aus der Gruppe ausgewählt ist, bestehend aus Cetyltrimethylammoniumbromid und Octadecyltrimethylammoniumsalz.

18. Verfahren nach einem der vorangegangenen Ansprüche, wobei die magnetischen Teilchen aus einem Material bestehen, welches aus der Gruppe ausgewählt ist, bestehend aus Eisen, Eisenoxid, Eisennitrid, Eisencarbid, Nickel und Kobalt und Gemischen und Legierungen davon.

19. Verfahren nach einem der vorangegangenen Ansprüche, wobei die magnetischen Teilchen paramagnetische Teilchen oder magnetische Latexteilchen sind.

20. Verfahren nach einem der vorangegangenen Ansprüche, wobei die magnetischen Teilchen einen mittleren Durchmesser im Bereich von etwa 0,1-100 µm haben.

21. Verfahren nach Anspruch 20, wobei die magnetischen Teilchen einen mittleren Durchmesser im Bereich von etwa 1-50 µm haben.

22. Verfahren nach Anspruch 21, wobei die magnetischen Teilchen einen mittleren Durchmesser im Bereich von etwa 1-5 µm haben.

23. Verfahren nach Anspruch 1 und irgendeinem davon abhängigen Anspruch, wobei die Protein enthaltenden Materialien aus der Gruppe ausgewählt sind, bestehend aus Zellen, Immunglobulinen, Proteinen und Enzymen.

24. Verfahren nach einem der vorangegangenen Ansprüche, wobei die hergestellte Probe für die Verwendung in einem Hybridisierungsassay geeignet ist.

25. Verfahren nach einem der vorangegangenen Ansprüche, wobei die magnetischen Teilchen beschichtet sind.

26. Verfahren nach einem der vorangegangenen Ansprüche, wobei die hergestellte Probe für die Verwendung in einem Immunoassay geeignet ist.

27. Kit, der geeignet ist, um Proben für die Verwendung in einem Assay herzustellen, und der folgendes umfaßt:
(a) einen Polyalkylenglycol,
(b) magnetische Teilchen, die in der Lage sind, unspezifisch an Protein enthaltende Materialien, die in den Proben enthalten sind, zu binden,
(c) Salz mit einer Konzentration von weniger als oder gleich etwa 0,2 M und
(d) Anweisungen für die Herstellung der Proben.

28. Kit nach Anspruch 27, wobei der Polyalkylenglycol Polyethylenglycol ist.

29. Kit nach Anspruch 27 oder 28, welcher weiterhin eine Pufferlösung umfaßt.

30. Kit nach Anspruch 27 oder 28, welcher weiterhin eine Lyselösung umfaßt.

31. Kit nach einem der Ansprüche 27 bis 30, wobei das Salz eine Konzentration von weniger als oder gleich etwa 0,125 M hat.

32. Kit nach Anspruch 31, wobei das Salz eine Konzentration im Bereich von etwa 0,01-0,10 M hat.

33. Kit nach einem der Ansprüche 27 bis 32, wobei das Salz aus der Gruppe ausgewählt ist, bestehend aus NaCl, MgCl₂, LiCl und KCl.

34. Kit nach einem der Ansprüche 27 bis 33, welcher weiterhin ein Detergens umfaßt.

35. Kit nach einem der Ansprüche 27 bis 34, wobei das Detergens ein kationisches Detergens ist.

36. Kit nach Anspruch 35, wobei das kationische Detergens ein quaternäres Ammoniumsalz ist.

37. Kit nach Anspruch 36, wobei das quaternäre Ammoniumsalz aus der Gruppe ausgewählt ist, bestehend aus Cetyltrimethylammoniumbromid und Octadecyltrimethylammoniumsalz.

38. Kit nach einem der Ansprüche 27 bis 37, wobei die magnetischen Teilchen aus einem Material bestehen, welches aus der Gruppe ausgewählt ist, bestehend aus Eisen, Eisenoxid, Eisennitrid, Eisencarbid, Nickel und Kobalt und Gemischen und Legierungen davon.

39. Kit nach einem der Ansprüche 27 bis 38, wobei die magnetischen Teilchen paramagnetische Teilchen oder magnetische Latexteilchen sind.

40. Kit nach einem der Ansprüche 27 bis 39, wobei die magnetischen Teilchen einen mittleren Durchmesser im Bereich von etwa 0,1-100 µm haben.

## Revendications

1. Procédé pour la préparation d'un échantillon destiné à être utilisé dans une analyse, comprenant les étapes consistant à :
(a) ajouter un polyalkylèneglycol et des particules magnétiques à un échantillon pour former un mélange, les particules magnétiques étant capables de se lier non spécifiquement à des matières contenant des protéines présentes dans le mélange, et le mélange ayant une concentration en sels inférieure ou égale à environ 0,2 M ;
(b) précipiter les matières contenant les protéines à partir du mélange lorsque les matières contenant des protéines se lient de manière non spécifique aux particules magnétiques, pour former un précipité et un surnageant ;
(c) rejeter le surnageant ; et
(d) isoler le précipité pour produire un échantillon préparé.

2. Procédé suivant la revendication 1, qui comprend en outre les étapes consistant à :
(e) dissoudre l'échantillon préparé dans une solution pour former un mélange de matières contenant des protéines non liées et des particules magnétiques ;
(f) appliquer un champ magnétique au mélange pour éliminer les particules magnétiques ;
(g) recueillir les matières contenant des protéines pour former un échantillon préparé isolé.

3. Procédé suivant la revendication 1 ou 2, dans lequel l'échantillon est un échantillon de tissu, un échantillon de sang entier, un échantillon de globules blancs, un échantillon de plasma, un échantillon de sérum, un échantillon de cellules en culture, un échantillon de matière fécale ou un échantillon d'urine.

4. Procédé pour la préparation d'un échantillon de sang destiné à être utilisé dans une analyse, comprenant les étapes consistant à :
(a) ajouter un polyalkylèneglycol et des particules magnétiques à un échantillon de sang pour former un mélange, les particules magnétiques étant capables de se lier non spécifiquement aux cellules présentes dans le mélange, et le mélange ayant une concentration en sels inférieure ou égale à environ 0,2 M ;
(b) précipiter les cellules à partir du mélange lorsque les cellules se lient non spécifiquement aux particules magnétiques, pour former un précipité et un surnageant ;
(c) rejeter le surnageant ; et
(d) isoler le précipité pour produire un échantillon de sang préparé.

5. Procédé suivant la revendication 4, qui comprend en outre les étapes consistant à :
(e) dissoudre l'échantillon de sang préparé dans une solution pour former un mélange de cellules sanguines non liées et de particules magnétiques ;
(f) appliquer un champ magnétique au mélange pour éliminer les particules magnétiques ;
(g) recueillir les cellules sanguines pour former un échantillon de sang préparé isolé.

6. Procédé suivant l'une quelconque des revendications précédentes, dans lequel l'étape de précipitation comprend en outre l'application d'un champ magnétique.

7. Procédé suivant l'une quelconque des revendications précédentes, dans lequel le polyalkylèneglycol est le polyéthylèneglycol.

8. Procédé suivant l'une quelconque des revendications précédentes, dans lequel la solution est une solution tampon.

9. Procédé suivant l'une quelconque des revendications précédentes, dans lequel la solution est une solution de lyse.

10. Procédé suivant l'une quelconque des revendications précédentes, dans lequel le mélange a une concentration en sels inférieure ou égale à environ 0,125 M.

11. Procédé suivant la revendication 10, dans lequel le mélange a une concentration en sels comprise entre 0,01 et 0,10 M.

12. Procédé suivant l'une quelconque des revendications précédentes, dans lequel le sel est choisi dans le groupe consistant en NaCl, MgCl₂, LiCl et KCl.

13. Procédé suivant l'une quelconque des revendications précédentes, dans lequel le mélange comprend en outre un détergent.

14. Procédé suivant l'une quelconque des revendications précédentes, dans lequel le détergent est présent en une concentration d'environ 1 à 10 mM.

15. Procédé suivant la revendication 13 ou 14, dans lequel le détergent est un détergent cationique.

16. Procédé suivant la revendication 15, dans lequel le détergent cationique est un sel d'ammonium quaternaire.

17. Procédé suivant la revendication 16, dans lequel le sel d'ammonium quaternaire est choisi dans le groupe consistant en le bromure de cétyltriméthylammonium et un sel d'octadécyltriméthylammonium.

18. Procédé suivant l'une quelconque des revendications précédentes, dans lequel les particules magnétiques sont constituées d'une matière choisie dans le groupe consistant en fer, oxyde de fer, nitrure de fer, carbure de fer, nickel et cobalt ainsi que leurs mélanges et alliages.

19. Procédé suivant l'une quelconque des revendications précédentes, dans lequel les particules magnétiques sont des particules paramagnétiques ou des particules de latex magnétiques.

20. Procédé suivant l'une quelconque des revendications précédentes, dans lequel les particules magnétiques ont un diamètre moyen dans l'intervalle d'environ 0,1 à 100 µm.

21. Procédé suivant la revendication 20, dans lequel les particules magnétiques ont un diamètre moyen dans l'intervalle d'environ 1 à 50 µm.

22. Procédé suivant la revendication 21, dans lequel les particules magnétiques ont un diamètre moyen dans l'intervalle d'environ 1 à 5 µm.

23. Procédé suivant la revendication 1 et n'importe quelle revendication qui en dépend, dans lequel les matières contenant des protéines sont choisies dans le groupe consistant en des cellules, des immunoglobulines, des protéines et des enzymes.

24. Procédé suivant l'une quelconque des revendications précédentes, dans lequel l'échantillon préparé est apte à l'utilisation dans une analyse d'hybridation.

25. Procédé suivant l'une quelconque des revendications précédentes, dans lequel les particules magnétiques sont enrobées.

26. Procédé suivant l'une quelconque des revendications précédentes, dans lequel l'échantillon préparé est apte à l'utilisation dans une analyse immunologique.

27. Kit utile dans la préparation d'échantillons destinés à être utilisés dans une analyse, comprenant :
(a) un polyalkylèneglycol ;
(b) des particules magnétiques qui sont aptes à la liaison non spécifique des matières contenant des protéines présentes dans les échantillons ;
(c) un sel à une concentration inférieure ou égale à environ 0,2 M ; et
(d) des instructions pour la préparation des échantillons.

28. Kit suivant la revendication 27, dans lequel le polyalkylèneglycol est le polyéthylèneglycol.

29. Kit suivant la revendication 27 ou 28, qui comprend en outre une solution tampon.

30. Kit suivant la revendication 27 ou 28, qui comprend en outre une solution de lyse.

31. Kit suivant l'une quelconque des revendications 27 à 30, dans lequel le sel a une concentration inférieure ou égale à environ 0,125 M.

32. Kit suivant la revendication 31, dans lequel le sel a une concentration dans l'intervalle d'environ 0,01 à 0,10 M.

33. Kit suivant l'une quelconque des revendications 27 à 32, dans lequel le sel est choisi dans le groupe consistant en NaCl, MgCl₂, LiCl et KCl.

34. Kit suivant l'une quelconque des revendications 27 à 33, qui comprend en outre un détergent.

35. Kit suivant l'une quelconque des revendications 27 à 34, dans lequel le détergent est un détergent cationique.

36. Kit suivant la revendication 35, dans lequel le détergent cationique est un sel d'ammonium quaternaire.

37. Kit suivant la revendication 36, dans lequel le sel d'ammonium quaternaire est choisi dans le groupe consistant en le bromure de cétyltriméthylammonium et un sel d'octadécyltriméthylammonium.

38. Kit suivant l'une quelconque des revendications 27 à 37, dans lequel les particules magnétiques sont constituées d'une matière choisie dans le groupe consistant en fer, oxyde de fer, nitrure de fer, carbure de fer, nickel et cobalt ainsi que leurs mélanges et alliages.

39. Kit suivant l'une quelconque des revendications 27 à 38, dans lequel les particules magnétiques sont des particules paramagnétiques ou des particules de latex magnétiques.

40. Kit suivant l'une quelconque des revendications 27 à 39, dans lequel les particules magnétiques ont un diamètre moyen dans l'intervalle d'environ 0,1 à 100 µm.
